# EUROPEAN PATENT APPLICATION

(11) **EP 3 772 536 A1**
(43) Date of publication of application: **10.02.2021**
(21) Application number: 19190534.8
(22) Date of filing: 07.08.2019
(51) Int. Cl.: C12N 1/02, C12N 1/12, C12M 1/00

(54) **METHOD AND SYSTEM FOR CONCENTRATING AN ALGAL SUSPENSION**

(71) Applicant: P3 B.V., 7861 TB Oosterhesselen (NL)
(72) Inventor: JAGER, Filips Gustaaf Hendrik, 7861 TB OOSTERHESSELEN (NL); VAN VELZEN, Dick, 2343 BR OEGSTGEEST (NL); HULSHOFF, Hendrik Jan, 8701 DV BOLSWARD (NL)
(74) Representative: van Dam, Vincent

(57) **Abstract**

The present invention relates to a method and a system for concentrating an algal suspension. The invention also relates to a method for cultivating algae which implements said method for concentrating an algal suspension and a method for producing algal products which implements said method for concentrating an algal suspension.

## Description

The present invention relates to a method and a system for concentrating an algal suspension. The invention also relates to a method for cultivating algae which implements said method for concentrating an algal suspension and a method for producing algal products which implements said method for concentrating an algal suspension.

### Introduction

Algae form a potent source for valuable products such as proteins and lipids and plastic precursors, which can be used as raw materials in food, oil and plastics industry.

The production of these products is a multistep process involving cultivation of the algae, harvest, and extraction of the desired products.

Harvesting algae requires concentration and dewatering of the algal biomass in order to allow extraction of the desired products, for instance by solvent extraction.

Concentration and dewatering of algae may also be desired if algae are to cultivated in a multistage process including a first stage of propagation and generation of biomass and second stage of production of algal products by the algae propagated in the first stage. The second phase necessitates a shift in metabolism, which can be induced by altering cultivation conditions, in order to allow production of algal products such as lipids, proteins and compounds such as polyhydroxy butyrate, which may serve as a raw material for plastics. This production requires that the algae are concentrated alive.

Conventional dewatering techniques include centrifugation, filtration (such as belt filtration, microfiltration, ultrafiltration, rotary filtration, cross-flow filtration, vacuum drum filtration) and direct drying (such as freeze drying and spray drying). These dewatering techniques have their drawbacks.

Centrifugation is energy intensive because algae need to be spun off fast due to the little difference in weight with the surrounding growth media. Because of the high centrifugal forces required, it is also stressful for the algae, which may have adverse effects on further process steps, including one or more further cultivation stages, because many algae will die during centrifugation. Centrifugation is also not easily scalable and requires high maintenance because it involves fast moving mechanical parts.

Filtration techniques often involve high operating costs for membrane replacement, clogging and pumping if operated on a large scale. In particular the formation of filter cake causes problems. Although less clogging and formation of filter cake occurs when using cross-flow filtration, the latter may only be used to dewater the algae to a limited amount. Moreover, cross-flow filtration is very expensive and not easily scalable.

Drying a wet algal suspension involves a large amount of energy and time so it is in general unsuitable for commercial purposes. Moreover, the algae will die in the process.

### Summary of the invention

The present invention aims to overcome the abovementioned drawbacks.

In a first aspect the invention relates to a method for concentrating an algal suspension, comprising using at least one device having at least two flow paths separated by a membrane, comprising i) introducing an algal suspension comprising a first concentration of algae into a first of said two flow paths at a pressure difference between said first flow path and a second of said flow paths which is sufficient to enable flow of water across the membrane from said first flow path to said second flow path, ii) discharging water entered in said second flow path from said second flow path, and iii) recovering the algae from said first flow path in a liquid medium to obtain an algal suspension which is more concentrated compared to the algal suspension introduced into said first flow path.

In a second aspect the invention relates to a system for producing a concentrated algal suspension, comprising a bioreactor configured to cultivate a first algal suspension, at least one device having at least two flow paths separated by a membrane wherein a first flow path on one side of said membrane is configured to receive said first algal suspension from said bioreactor, means to apply a pressure difference over said membrane to enable flow of water across the membrane to a second of said flow paths; and means to recover algae from said first flow path as a second algal suspension which is more concentrated than said first algal suspension.

In a third aspect the invention relates to a method for cultivating algae in a multistage process, which comprises cultivation of algae in at least two separate cultivation stages, comprising a1) a first cultivation stage wherein algae are cultivated under first conditions which allow propagation of the algae to provide a first algal suspension; b1) concentrating said first algal suspension in accordance with the method of the first aspect to obtain a second algal suspension which is more concentrated than said first algal suspension; a2) a second cultivation stage wherein said second algal suspension is cultivated under second conditions which differ from the first conditions to provide a third algal suspension.

In a fourth aspect the invention relates to a method for producing algal products comprising: a) cultivation of algae in an aqueous culture medium to provide an algal suspension; b) concentrating said algal suspension in accordance with the method of the first aspect of the invention to obtain an algal suspension which is more concentrated compared to the algal suspension of a); and c) extracting algal products from the concentrated algal suspension obtained in b).

### Short description of the drawings

Fig. 1 shows a schematic representation of an exemplary device for use in accordance with an embodiment of the method of the invention, wherein algae are introduced into the device and accumulated on the membrane of the device.
Fig. 2 shows a schematic representation of the device of fig. 1 wherein accumulated algae are removed from the membrane of the device.
Fig. 3 shows a schematic representation of a device for use in accordance with another embodiment of the method of invention.
Fig. 4 shows a perspective view of the device of figures 1, 2 and 3.
Fig. 5 shows a schematic overview of an exemplary system according to the invention.

### Detailed description of the invention

The present invention is based on the finding of the inventors that a device having at least two flow paths separated by a membrane can be used in a process and system for concentrating algae in an aqueous suspension by applying a pressure difference over the membrane, so that a concentrated algal suspension can be obtained in a non-destructive way. In the context of the invention a concentrated algal suspension is to be understood as containing more grams of algae per liter of the suspension than the initial suspension before concentrating the algae. The terms concentrating and dewatering are used interchangeably in this application, because they both relate to obtaining an algal suspension having more grams of algae per liter suspension compared to an initial algal suspension.

The process and system according to the invention require low energy input and allow for easy upscaling because as many devices as desired for the required throughput can be arranged in parallel fashion. It is therefore preferred that the system according to the invention comprises a plurality of said devices in parallel.

A suitable membrane for use in the context of the invention may have pores having diameter sizes between 0,001 µm and 0,1 µm, such as 0,03 µm. These allow transport of water and small molecules across the membrane while algae are not capable to pass. The specific pore size may be determined based on the size of the algae to be treated, because various algal species may vary in size.

A pressure difference of 0,5 bar has been found to be sufficient to achieve flow of water across the membrane. Nevertheless the pressure may be lowered or increased, for instance depending on the pore size of the membrane or the size of the algae to be treated.

The present invention is suitable to dewater and concentrate an algal suspension. The algae in the algal suspension may comprise broken algae and intact algae, i.e. dead and living algae.

In a preferred embodiment said device comprises a plurality of capillary membranes each having an inside of the capillary membrane and an outside of the capillary membrane; wherein abovementioned stage i) comprises introducing said first algal suspension into the inside of the capillary membrane; and abovementioned stage ii) comprises discharging said water from the outside of said capillary membrane. In this respect it is preferred in the system of the invention that said at least one device comprises a plurality of capillary membranes each having an inside of the capillary membrane and an outside of the capillary membrane. This allows to introduces treatment of a flow of algal suspension for a prolonged period of time and for larger volumes. Such a setup allows easy circulation of algae and arrangement of multiple devices in parallel fashion.

The membranes in the device are preferably inert hollow fibers, for instance made of artificial polymers, including cellulose acetate, polysulfone, polyethersulfone, and polyvinylidene fluoride. Such membranes are commonly used in dialyzers. A device in the context of the invention may therefore suitably be a dialyzer, such as a hollow fiber dialyzer. The walls of the hollow fiber dialyzer have pores, so that the walls function as a membrane in the context of the invention.

Surprisingly hemo-dialyzer type dialyzers have been found to be very suitable for purposes of concentrating and dewatering algal suspensions in accordance with the invention. In a "traditional" setup of a hemo-dialyzer blood is caused to circulate in capillaries between an inlet and an outlet, while a dialysate is also caused to circulate between another inlet and another outlet, wherein the dialysate and blood are separated by the walls of the capillaries which form membranes over which urea molecules and other small molecules from the circulating blood can flow to the passing dialysate. This way the blood is cleaned of urea while the blood cells and larger molecules can be circulated back to the patient's body. These dialyzers can be used in the method and system of the invention using a "modified" setup. A dialyzer in the context of the invention may suitably be a hemo-dialyzer type dialyzer. The present invention allows reuse of hemo-dialyzers which are discarded from medical environments such as hospitals. This saves considerable costs and is environmental-friendly. Furthermore, dialyzers are well configured to be arranged in a parallel fashion, thereby allowing a high throughput of algae.

The use of a dialyzer has also advantages in terms of versatility. In other words, it can be used in different "modified" setups which allow dewatering of algae as will be explained below.

In one embodiment introducing said algal suspension comprises introducing said algal suspension under a sufficient pressure to enable flow of water across the membrane from the first flow path to said second flow path, and retaining said algae in said first flow path in this introduction stage, and wherein said introducing of the algal suspension results in accumulation of algae on a first side of said membrane; and wherein the method comprises subsequent recovering of the accumulated algae from said first side of the membrane which comprises releasing the accumulated algae from said first side of said membrane with a liquid medium in an amount less than the amount of water flowed across the membrane in said introduction stage to obtain an algal suspension which is concentrated compared to the algal suspension introduced into said first flow path. For this purpose, said means to apply a pressure difference over said membrane to allow flow of water across the membrane to said second flow path in the system of the invention may be a means for introducing said first algal suspension into the first flow path under pressure, such as a pump.

In this embodiment accumulation of algae is used advantageously to recover and obtain a concentrated algal suspension. In this embodiment no feed of liquid from outside the device into the second flow path takes place during introduction of the algae and flow of water across the membrane into the second flow path. In other words the only liquid flowing through the second flow path during introduction of the algal suspension is the water extracted from the algae which were introduced in the first flow path. For this purpose an algal suspension comprising a first amount of water is introduced into a first of said two flow paths on a first side of said membrane at a pressure sufficient to enable flow of water across the membrane to a second side of said membrane. Due to this pressure water escapes from the algal suspension across the membrane, where it enters the second flow path. The water in the second flow path flows out of the dialyzer and can be discharged, for instance into a water reservoir. The applied pressure and the escape of water from the first suspension results in accumulation of algae on said first side of said membrane and introduction of water in said second flow path. The algae accumulated on said first side of said membrane are then recovered with water or another liquid medium, such as a salt solution, in an amount less than said first amount to obtain an algal suspension which is concentrated compared to the algal suspension introduced into the first flow path. This way the accumulated biomass can be recovered with a minimal amount of water or another liquid medium required to release the accumulated biomass and allow flow of the resulting concentrated algal suspension out of the device to a location of downstream processing. As a result this embodiment allows to concentrate the biomass to a very high concentration. In fact, the inventors have observed that this setup allows concentration by extracting water up to 80% or more of the volume of the suspension entered into the device.

If for this embodiment a multi-capillary type dialyzer, such as a hemo-dialyzer type dialyzer, is used, this requires a "modified setup" of the mode of action compared to its "traditional setup". In this case the first algal suspension enters the capillaries of the dialyzer, which serve in this case as a first flow path in the context of the invention, from one or both of the inlet and the outlet (i.e. the blood inlet and outlet in terms of traditional use for blood dialysis). If it enters from one of the inlet and outlet, the other one is closed. In other words, the only way water can go in this setup during the phase of introduction of the first algal suspension is across the walls of the capillaries to the exterior of the capillaries. The exterior of the capillaries then functions as second flow path in the context of this invention. The water can then be discharged from one or both of the inlet and outlet for liquid outside the capillaries (i.e. the dialysate inlet and outlet in terms of traditional use for blood dialysis). It is also possible to reverse this setup; in this case the first algal suspension is introduced into the flow path on the exterior of the capillaries via one or both of the inlet and outlet of this flow path and the extracted water is discharged from one or both of the inlet and outlet of the capillaries. In this reversed setup the exterior of the capillaries then functions as first flow path in the context of the invention and the interior of the capillaries then functions as second flow path.

It is preferred that the accumulated algae are recovered in an aqueous medium such as water to obtain a more concentrated algal suspension compared to the algal suspension introduced into the device. This way the algae may be kept alive which is advantageous for certain purposes wherein it is desired that the algae are alive. This is in particular the case if the algae are cultivated in a process for production of algal products in a two-stage process, wherein the first stage involves autotrophic algal growth to create biomass and wherein the second stage involves production of valuable metabolites by the algae under different conditions (for example mixotrophic propagation), including the addition of nutrients and possibly sugars to the algal suspension. Production of valuable metabolites requires multistage propagation, comprising at least two stages wherein in the second phase different conditions and high concentrations of living algae are required. Therefore, before entering the second stage of propagation (which can be mixotrophic) the algae have to be concentrated, i.e. dewatered partially, whilst being kept alive. In such a two phase process it is therefore essential that the algae are kept alive after the first autotrophic propagation stage and thus during the concentration of the algal suspension. Moreover, by using water no additional steps of washing the device will be required. It is therefore preferred that the algae are recovered with water in an amount less than said first amount to obtain an algal suspension which is concentrated compared to the initial algal suspension. This water may conveniently be water containing a suitable concentration of salts and nutrients, for instance for creating mixotrophic conditions or a part of the water extracted from the first algal suspension. In this respect recovering the algae accumulated on said first side of said membrane may suitably be performed with water in or discharged from said second flow path. This ensures minimal use of water in the process.

Recovering the algae accumulated on said first side of said membrane may suitably be performed by reversing the flow, i.e. by introducing water under pressure in the second flow path. This results in a flow of water across the membrane from the side of the second flow path to the first flow path, causing agitation on the membrane surface at the side of the first flow path. This causes the accumulated algae to be released from the membrane on the side of the first flow path into the water entering the side of the first flow path so that the algae are resuspended and can be recovered. This allows easy and water-efficient recovery of the algae. It is therefore preferred that recovering the algae accumulated on said first side of said membrane comprises passing water through said second flow path at a pressure sufficient to enable flow of water across the membrane to the first flow path, for instance by reversing the flow of the discharged water, thereby releasing algae from the membrane to obtain said algal suspension which is concentrated compared to the algal suspension introduced into said first flow path. In this case the water entered into and/or discharged from said second flow path is used to release and resuspend the accumulated algae. Because the amount of water used to recover the accumulated algae from the membrane is less than the amount extracted from the algal suspension during introduction, this leads to an algal suspension which contains less water compared to the algal suspension introduced into the first flow path. In other words the recovered suspension contains more grams of algae per liter suspension than the suspension introduced, i.e. the recovered suspension is more concentrated. Reverse of flow and resuspending accumulated algae can be effected easily by providing the system of the invention with means configured to reverse flow of water from said second flow path back to the first flow path to allow release of accumulated algae from said first side of the membrane in the first flow path. Such means comprise means configured to reverse the flow of extracted water back into the second flow path, such as valves and pumps.

For purposes of release of the accumulated algae it is preferred that the membrane is made of artificial, smooth and inert polymers, such as cellulose acetate, polysulfone, polyethersulfone, and polyvinylidene fluoride. Because these hollow fibers are smooth and inert only a very little amount of water is required in order to effect release of accumulated algae from the membrane. This on its turn enables high dewatering and concentration of the algal suspension. If for this purpose hollow fiber dialyzers are used it is preferred that the first algal suspension is introduced into the hollow fibers. In other words, in this case the inside of the hollow fibers constitute said first flow path. It is further preferred that the dialyzer is a hollow fiber dialyzer and the hemo-dialyzer type.

In another embodiment the method according to the invention comprises passing said first algal suspension into an inlet of said first flow path towards an outlet of said first flow path, applying suction onto said second flow path to effect flow of water from said first flow path across the membrane to said second flow path, thereby extracting water from said algal suspension during its passage through said first flow path, so as to obtain said algal suspension which is concentrated compared to the algal suspension introduced into said first flow path. In this embodiment a negative pressure is created by applying suction force onto the second flow path. The algal suspension runs through the first flow path along the flow path from inlet to outlet and during its passing through it water is extracted from the suspension and flows through the membrane into the second flow path as a result of the suction force. As a result the flowing algal suspension at the outlet side contains less water and is a more concentrated algal suspension compared to the algal suspension introduced on the inlet side. For this purpose, said means to apply a pressure difference over said membrane to allow flow of water across the membrane to said second flow path can be provided as a means for applying suction in the second flow path, such as a steady flow pump. This embodiment allows very fast concentration by extracting water up to 50% of the volume of the suspension entered into the device. Such a dewatering rate has been observed to be sufficient for a subsequent second cultivation step in which the algae are cultivated under conditions that allow production of algal products (for example mixotrophic conditions) and because this embodiment can effect dewatering in such a fast rate, almost all cells remain intact and available for production of valuable metabolites. This embodiment is therefore very suitable to concentrate and dewater a living algal culture in a non-destructive manner.

If desired the concentrated algal suspension can be recirculated to the inlet of the device for further concentration until a desired algae concentration has been effected. This setup can be performed in an almost continuous fashion.

If for this embodiment a multi-capillary type dialyzer, such as a hemo-dialyzer type dialyzer, is used, this can be done using the following modified setup. In this case the first algal suspension enters the capillaries of this dialyzer, which serve in this case as a first flow path in the context of the invention, from the inlet of the capillaries and it leaves the dialyzer at the outlet of the capillaries (i.e. the blood inlet and outlet in terms of traditional use for blood dialysis) . The exterior of the capillaries then functions as second flow path in the context of this invention in which water extracted from the algal suspension is discharged by suction from one or both of an inlet side and an outlet side for liquid outside the capillaries (i.e. the dialysate inlet and outlet in terms of traditional use for blood dialysis). It is also possible to reverse this setup; in this case the first algal suspension is introduced into the flow path on the exterior of the capillaries via an inlet for this flow path and concentrated algal suspension is discharged from an outlet of the flow path exterior of the capillaries. The suction in this case is applied on one or both of the inlet and outlet of the capillaries so that the water extracted from the algae can leave the dialyzer from one or both of the inlet and outlet of the capillaries. In this reversed setup the exterior of the capillaries then functions as first flow path in the context of the invention and the interior of the capillaries then functions as second flow path.

The algal suspension introduced into the first flow path may be an algal culture directly drawn from a bioreactor, but it may also be an algal suspension which has already been subjected to earlier dewatering processes such as flocculation or flotation or an earlier dewatering process in accordance with the invention.

As mentioned above the present invention can be applied in a multistage process which comprises at least two separate cultivation stages, wherein the first stage involves algal propagation to create biomass and wherein the second stage involves production of valuable metabolites by the algae under different conditions than the conditions for the first cultivation stage. The invention therefore also relates to a multistage cultivation process which comprises cultivation of algae in at least two separate cultivation stages, comprising a1) a first stage wherein algae are cultivated under first conditions which allow propagation of the algae to provide a first algal suspension; b1) concentrating said first algal suspension in accordance with the method of any of the previous claims to obtain a second algal suspension which is more concentrated than said first algal suspension; a2) a second stage of cultivation wherein said second algal suspension is cultivated under second conditions which differ from the first conditions to provide a third algal suspension.

In the cultivation process of the invention the first cultivation stage is aimed at propagation, i.e. cell division of the algae so as to create biomass. This is preferably performed under autotrophic conditions, under which the only carbon source supplied to the algae is CO₂ and algal propagation takes place under conditions that allow photosynthesis. During autotrophic algal growth in principle the only carbon source added is CO₂ and this phase primarily functions to achieve a high number of living algal cells.

It is however desired that the algae produce valuable metabolites, such as proteins, lipids and polymeric biomolecules as PHA's and the like. This requires alteration of algal metabolism and thus different cultivation regimes. In the abovementioned second cultivation stage the algae are subjected to cultivation conditions which allow production of algal products, in other words the algae are forced into the production mode. This usually includes nitrogen deprivation and if needed mixotrophic conditions in which additional carbon sources are added, for instance sugars. Moreover, it is desirable that the algae are as concentrated as possible and alive to allow optimal use of nutrients. The more concentrated the algae are, the less valuable nutrients will be wasted. It is therefore desirable in a multistage algal cultivation process that the algae are concentrated after the first stage before entering the second propagation stage. This allow allows to shift the full algal colony into the production mode, which is advantageous for production efficiency. This concentration step can conveniently be performed with the method of the first aspect of the invention concentrating an algal suspension.

It is in this respect preferred in a multistage cultivation process that step b1) comprises passing said first algal suspension into an inlet of said first flow path towards an outlet of said first flow path, applying suction onto said second flow path to enable flow of water from said first flow path across the membrane to said second flow path, thereby extracting water from said algal suspension during its passage through said first flow path, so that said second algal suspension is obtained. The inventors have found that concentrating an algal suspension applying suction on the second flow path to extract water from a flowing algal suspension in accordance with the embodiment of the first aspect of the invention as described above puts very limited stress on the algae passing the first flow path. Therefore, this way of concentrating the algal suspension results in concentration of the algal suspension up to 50% with hardly any loss of algal production capacity due to algal death.

After the second cultivation stage it is preferred that the algae are further concentrated to allow efficient extraction of the produced valuable metabolites. This can be suitably be done with the concentration method of the first aspect of the invention. It is therefore preferred to apply a step b2) of concentrating said third algal suspension in accordance with the method of the first aspect of the invention to obtain a fourth algal suspension which is more concentrated than said third algal suspension.

The second water extraction/concentration step b2) may be performed in accordance with step b1). However, this second water extraction does not always require very mild conditions because the algae will be subjected to extraction and isolation of algal products after that, which involves the eventual death of the algae. The inventors have found that the embodiment wherein an algal suspension is forced to be retained in said first flow path during the stage of introduction of the algae into said first flow path, and wherein said introducing of the algal suspension at a pressure difference results in accumulation of algae on a first side of said membrane and flow of water from the first flow path to the second flow path allows to concentrate the algae quickly, concentrating the algae with 80% or more, which renders the algae ready for downstream processes as drying and extraction and isolation of desired products. It is therefore preferred that step b2) comprises introducing said algal suspension into said first flow path in an introduction stage during which algae are forced to be retained in said first flow path, and wherein said introducing of the algal suspension at said pressure difference results in accumulation of algae on a first side of said membrane and introduction of water in said second flow path; and wherein recovering the accumulated algae from said first side of the membrane is performed in a subsequent stage which comprises releasing the accumulated algae from said first side of said membrane with a liquid medium in an amount less than the amount of water flowed across the membrane in said introduction stage to obtain an algal suspension which is concentrated compared to the algal suspension introduced into said first flow path.

For performing the above described two-phase cultivation method and to allow concentration of algal suspensions in accordance with the invention, it is preferred to use separate bioreactors for the separate stages, because this allows a continuous multistage cultivation process. Therefore it is preferred that the system according the invention is configured with a first bioreactor configured to cultivate said first algal suspension, at least one first device having at least two flow paths separated by a membrane, wherein a first flow path on one side of said membrane is configured to receive said first algal suspension from said first bioreactor, means to apply a pressure difference over said membrane of said at least one first device to allow flow of water across the membrane to a second of said flow paths; and means to recover algae from said first flow path of said at least one first device as a second algal suspension which is more concentrated than said first algal suspension; a second bioreactor configured to receive said second algal suspension from said at least one first device, and which is further configured to cultivate said second algal suspension under different conditions than in said first bioreactor to provide a third algal suspension; at least one second device having at least two flow paths separated by a membrane, wherein a first flow path on one side of said membrane is configured to receive said third algal suspension from said second bioreactor, means to apply a pressure difference over said membrane of said at least one second device to allow flow of water across the membrane to a second of said flow paths; and means to recover algae from said first flow path of said at least one second device as a fourth algal suspension which is more concentrated than said third algal suspension. This fourth algal suspension may then be used for step c), extracting the algal products, such as lipids, proteins and bioplastic precursors.

Culturing algae can be performed in any setup known in the art, for instance in a bioreactor.

The concentrated and dewatered algae obtained via the process of dewatering algae according to the first aspect of the invention or via the cultivation process of the third aspect of the invention can be used for downstream processing in order to extract and isolate the desired metabolic products from the algae (e.g. biopolymers, lipids, proteins and the like). In this respect the invention also relates to a method for producing algal products comprising: a) cultivation of algae in an aqueous culture medium to provide an algal suspension; b) concentrating said algal suspension in accordance with the method of the first aspect of the invention to obtain an algal suspension which is more concentrated compared to the algal suspension of a); and c) extracting algal products from the concentrated algal suspension obtained in b). In a preferred embodiment step c) is applied on an algal suspension obtained in accordance with the multistage cultivation method according to the third aspect of the invention. The algal products to be extracted and isolated may be selected from the group of lipids, proteins, including phycocyanin (such as C-phycocyanin), bioplastic precursors and other polymers, including polyhydroxy alkanoates (PHA) (such as polyhydroxy butyrate (PHB)), which are bioplastic precursors. Lipids and proteins can for instance be used in food industry. Algal lipids can be used as a source for biofuels, such as biodiesel. Other valuable products are polyhydroxy butyrate (PHB), which can be used as a raw material for plastics.

Extracting said algal products may also involve conventional techniques, including solvent extraction. Before extraction of the algal products the concentrated algal suspension may be subjected to further concentration steps or drying if necessary.

### Detailed description of the drawings

The invention will now be further elucidated in the attached drawings. The following explanation is meant to illustrate and explain the invention and not to limit the claims.

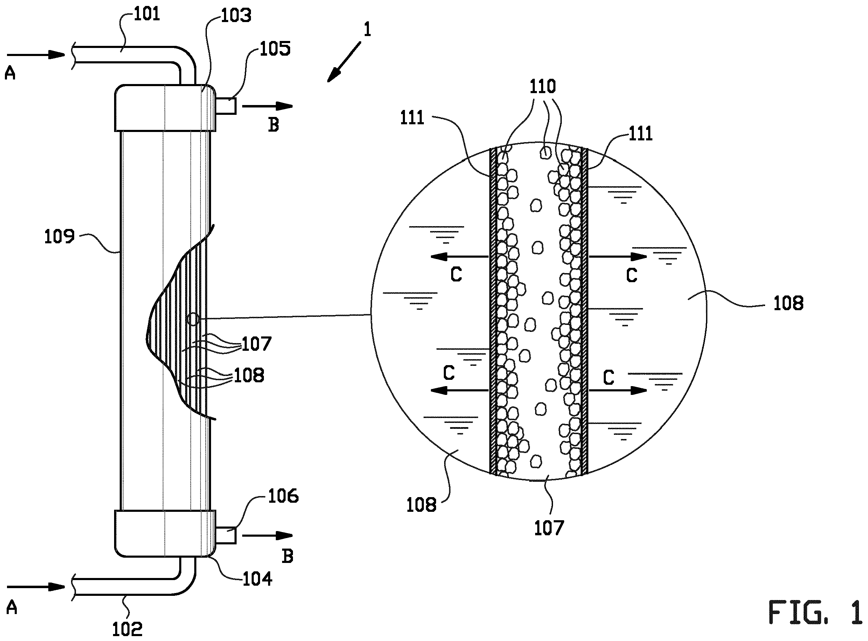

Figs. 1 to 4 show how a hollow fiber dialyzer 1 of the hemo-dialyzer type can be used to concentrate an algal suspension.

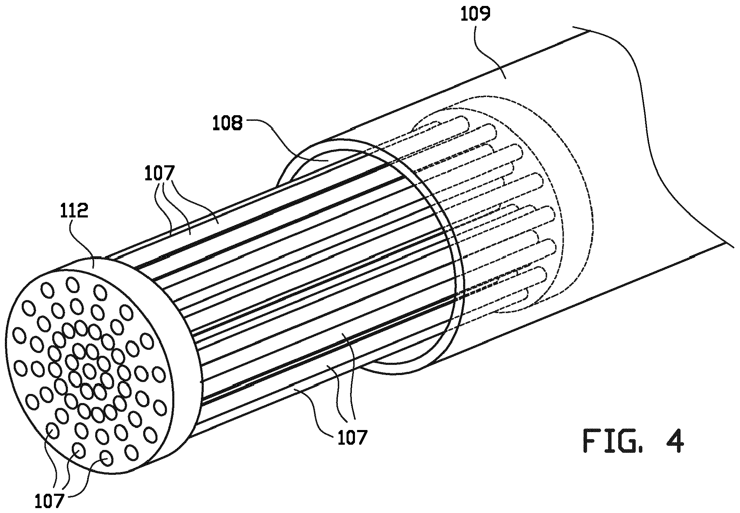

The hollow fiber dialyzer 1 has an outside body 109 in which a hollow fiber assembly 112 is enclosed between end caps 103 and 104. Said hollow fiber assembly is shown in detail in Fig.4. The hollow fibers 107 have walls 111 that are porous membranes with pores between 0,001 µm and 0,1 µm in diameter, such as 0,03 µm. These allow transport of water and small molecules across the membrane while algae are not capable to pass. Liquid may enter and/or exit the hollow fibers 107 of dialyzer 1 via tube 101 and tube 102. On the other hand liquid on the exterior 108 of the hollow fibers 107 may enter and/or exit the dialyzer 1 via tube 105 and 106.

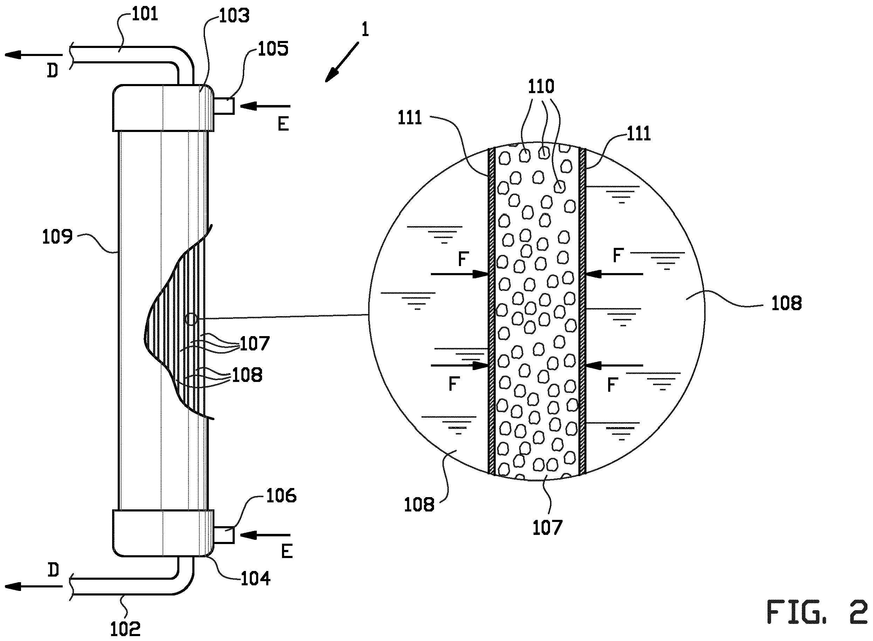

In a first setup dialyzer 1 is used as shown in Fig. 1 and 2. In this embodiment Fig. 1 shows how a first suspension of algae 110 is introduced into the hollow fibers 107 via tubes 101 and 102 (in the direction of arrows A), which in this setup both act as inlets for the algal suspension during introduction of said first algal suspension. In this case the first algal suspension enters the hollow fibers 107 of this dialyzer 1, which hollow fibers serve in this case as a first flow path in the context of the invention. Because the algal suspension in introduced into the hollow fibers under pressure from both tubes 101 and 102 the algal suspension is forced to be retained in the hollow fibers 107, and the only way water can go in this setup during the phase of introduction of the first algal suspension is across the walls 111 of the hollow fibers to the exterior 108 (see arrows C). These porous walls function as membrane. The exterior 108 of the hollow fibers 107 then functions as second flow path in the context of this invention for the water extracted from the algal suspension. During introduction of the algae 110 and flow of water across the membrane 111 into the exterior 108 no feed of liquid from outside the dialyzer 1 into the exterior flow path 108 takes place. The water can then be discharged from tubes 105 and 106 (in the direction of arrows B). After a certain period of introduction of algae 110 into dialyzer 1 a considerable amount of algae will be accumulated on the membranes 111 on the inside of the hollow tubes 107. This will eventually lead in blocking of the pores of the membrane 111.

At this moment the algae 110 accumulated on said membrane 111 are recovered by reversing the flow of water through the membrane 111 as shown in Fig. 2 with arrows F. To effect flow of water from the exterior side of the membrane water to the inner side of the fibers, water is returned to the exterior 108 of the hollow fibers via tubes 105 and 106 (in the direction of arrows E). This is done with a pressure sufficient to enable flow of water across the membrane to the inside of the hollow fibers 107, resulting in agitation on the inside of the hollow fibers and thereby releasing algae 110 from the membrane 111 to obtain a second algal suspension, which is more concentrated than the initial suspension introduced in dialyzer 1, because resuspending only requires a low amount of water. The concentrated algal suspension can then be recovered via tubes 101 and/or 102 in the direction of arrows D.

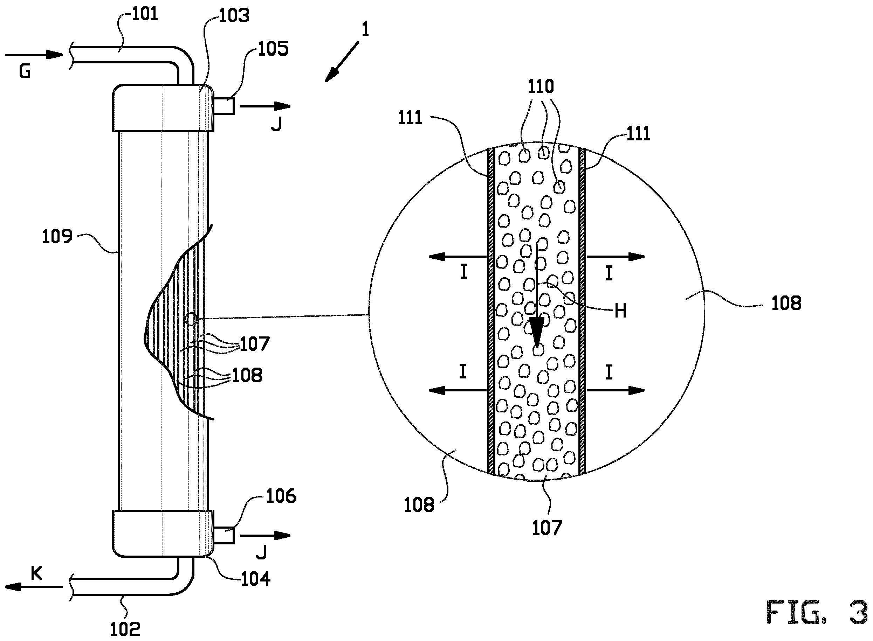

In a second setup dialyzer 1 is used as shown in Fig. 3. In this embodiment a suspension of algae 110 is introduced under pressure into the hollow fibers 107 via tube 101 (in the direction of arrow G). The algae 110 are allowed to flow through the hollow fibers in the direction of arrow H. On the other hand a pressure difference is effected across the membrane by creating a negative pressure in the exterior 108. This results in suction of water over membrane 111 in the direction of arrows I. The water extracted from the algae leaves the dialyzer via tubes 105 and/or 106 (in the direction of arrows J). This has the effect that the algal suspension passing hollow fiber 108 loses partially dewaters and concentrates in a downstream fashion during its passing through hollow fiber 108. The partially dewatered and thus concentrated algal suspension leaves the hollow fibers 107 and the dialyzer 1 via tube 102 (in the direction of arrow K) .

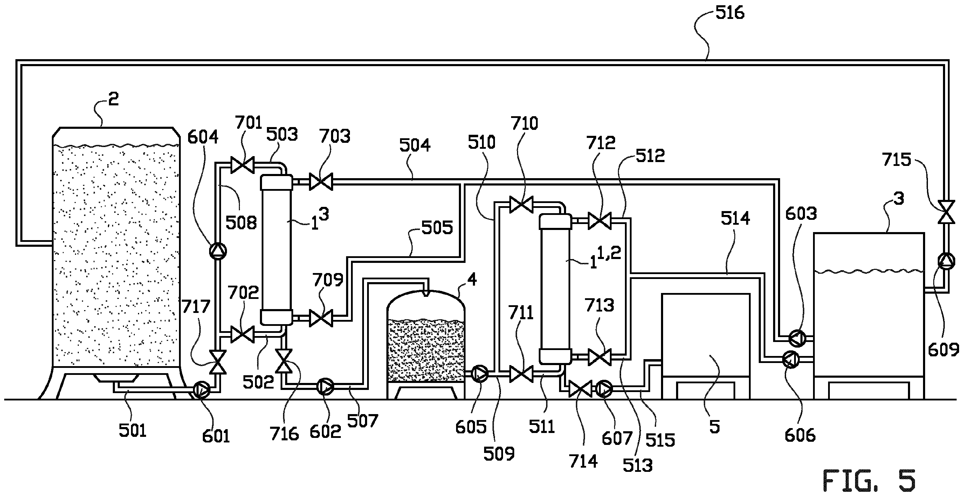

Fig. 5 shows a schematic overview of an exemplary system according to the invention. The system is configured for production of algal products in a two-stage process. In this exemplary embodiment the first stage involves autotrophic growth with CO₂ as the sole carbon source. The second stage involves production of valuable metabolites by the algae under different cultivation conditions.

For the first, autotrophic stage this system comprises a first bioreactor 2 in which a first algal suspension is cultured under autotropic conditions. In order concentrate this algal suspension whilst keeping them alive for the second stage, the algal suspension from bioreactor 2 is passed in a first concentration step via tube 501, 508, 503 to dialyzer 1³. Supply of said algal suspension to dialyzer 1³ can be regulated by means of pumps 601, 604 and valves 701, 702, 717. Dialyzer 1³ is configured and operated as described for Figs. 3. In other words, an algal suspension is passed through the hollow fibers from an inlet side to an outlet side of dialyzer 1³, while water is extracted over the porous walls of the hollow fibers by suction, effected with pump 603. Water extracted from the algal suspension is discharged from dialyzer 1³ to a water tank 3 via tubes 504 and/or 505. Control of flow and suction via tubes 504 and 505 can be is realized with help of pump 603 and valves 703, 709. In order to allow several rounds of water extraction the algal suspension can be recirculated from the outlet of the dialyzer 1³ to the inlet of dialyzer 1³ via lines 502, 508, 503 using pump 604. This allows further concentration and dewatering of the algal suspension in multiple cycles. The algal suspension is concentrated in this stage to a predetermined concentration which allows optimal multiphase cultivation and production of valuable metabolites requiring minimal amounts of nutrients and minimal addition of extra carbon in the form of sugars.

In order to allow multiphase cultivation the concentrated suspension which contains living algae is then passed to a second bioreactor 4 via tube 507 and with help of pump 602 and valve 716. In bioreactor 4 algae are exposed to a different propagation regimes in which additional carbon, such as sugars are supplemented, combined with alterations in conditions such as nitrogen deprivation. This forces the algae to produce glycogen and valuable metabolites such as polyhydroxy butyrate and lipids.

After a period of second phase cultivation said valuable metabolites will have to be extracted. This requires dewatering of the suspension to maximal levels, to obtain an algal suspension with a minimal amount of water. This can be effected by passing the algal suspension from bioreactor 4 via tubes 509, 510, 511 to a further dialyzer 1^{1,2} in accordance with the invention, which is configured and operated as described for Fig.1 and Fig.2. Flow of algae to dialyzer 1^{1,2} from bioreactor 4 is controlled with pump 605 and valves 710, 711. The algal suspension enters the hollow fibers of dialyzer 1^{1,2} via tubes 510, 511 under a pressure sufficient to allow flow of water from the algal suspension over the walls of the hollow fibers. During introduction of the algae 110 no feed of liquid from outside the dialyzer 1^{1,2} into the exterior of the hollow fibers takes place, so that water entering the exterior of the hollow fibers can then be discharged from dialyzer 1^{1,2} via tubes 512, 513, 514 to water tank 3. After a certain period of introduction of algae 110 into dialyzer 1^{1,2} the algae will accumulate on the porous walls on the inside of the hollow tubes of dialyzer 1^{1,2}. In order to realize release of algae accumulated in the hollow fibers of dialyzer 1^{1,2} the flow through tubes 512, 513, 514 is reversed towards dialyzer 1^{1,2}, controlled with valves 712, 713 and pump 606 so that water is forced over the membranes of the dialyzer into the interior of the hollow fibers thereof, thereby causing agitation on the inside of the hollow fibers and releasing algae from the membranes to obtain a further concentrated algal suspension, which contains a higher concentration of algae compared to the algal suspension introduced into dialyzer 1^{1,2}. Only a small amount of water is required for this. This further concentrated algal suspension is passed to means for lysing algae 5 via tube 515, using pump 607 and valve 714. Here the algae can be lysed, after which valuable algal products such as lipids, proteins and polyhydroxy butyrate can be extracted and isolated. If desired water recovered in water tank 3 can be recycled to bioreactor 2 via tube 516 using pump 609 and valve 715 in order to serve as growth medium for the algae. This enables recycling of possibly remaining nutrients in the water extracted from the algal suspensions.

## Claims

1. A method for concentrating an algal suspension, comprising using at least one device having at least two flow paths separated by a membrane, comprising
i) introducing an algal suspension comprising a first concentration of algae into a first of said at least two flow paths at a pressure difference between said first flow path and a second of said flow paths which is sufficient to enable flow of water from said first flow path to said second flow path,
ii) discharging water entered in said second flow path from said second flow path, and
iii) recovering the algae from said first flow path in a liquid medium to obtain an algal suspension which is more concentrated compared to the algal suspension introduced into said first flow path.

2. The method according to claim 1, wherein introducing said algal suspension comprises introducing said algal suspension under a sufficient pressure to enable flow of water across the membrane from the first flow path to said second flow path, and retaining said algae in said first flow path, and wherein said introducing of the algal suspension results in accumulation of algae on a first side of said membrane; and wherein the method comprises subsequent recovering of the accumulated algae from said first side of the membrane which comprises releasing the accumulated algae from said first side of said membrane with a liquid medium in an amount less than the amount of water flowed across the membrane during introduction of the algae into said first flow path to obtain an algal suspension which is concentrated compared to the algal suspension introduced into said first flow path.

3. The method according to claim 1 or 2, wherein recovering the algae accumulated on said first side of said membrane is performed by reversing the flow of water through the membrane to effect flow of water from the second flow path to the first flow path, resulting in release of accumulated algae from said first side of the membrane.

4. The method for concentrating an algal suspension according to claim 1, comprising passing said first algal suspension into an inlet of said first flow path towards an outlet of said first flow path,
applying suction onto said second flow path to effect flow of water from said first flow path across the membrane to said second flow path, thereby extracting water from said algal suspension during its passage through said first flow path, so as to obtain said algal suspension which is concentrated compared to the algal suspension introduced into said first flow path.

5. The method according to any of the previous claims, wherein said at least one device comprises a plurality of capillary membranes each having an inside of the capillary membrane and an outside of the capillary membrane;
wherein stage i) comprises introducing said first algal suspension into the inside of the capillary membrane; and
wherein stage ii) comprises discharging said water from the outside of said capillary membrane,
preferably wherein said capillary membranes are hollow fibers with porous walls, wherein said porous walls function as said membranes.

6. The method according to claim 4 or 5, wherein said at least one device is a hemo-dialyzer, preferably a hemo-dialyzer discarded from medical use.

7. A method for cultivating algae in a multistage process, which comprises cultivation of algae in at least two separate cultivation stages, comprising
a1) a first cultivation stage wherein algae are cultivated under first conditions which allow propagation of the algae to provide a first algal suspension;
b1) concentrating said first algal suspension in accordance with the method of any of the previous claims to obtain a second algal suspension which is more concentrated than said first algal suspension;
a2) a second cultivation stage wherein said second algal suspension is cultivated under second conditions which differ from the first conditions to provide a third algal suspension.

8. The method according to claim 7, wherein step b1) comprises passing said first algal suspension into an inlet of said first flow path towards an outlet of said first flow path, applying suction onto said second flow path to enable flow of water from said first flow path across the membrane to said second flow path, thereby extracting water from said algal suspension during its passage through said first flow path, so that said second algal suspension is obtained.

9. The method for cultivating algae according to claim 7 or 8, wherein said second conditions comprise nitrogen deprivation.

10. The method according to any of the claims 7 to 9, which comprises after stage a2), a step of:
b2) concentrating said third algal suspension in accordance with the method of any of the claims 1 to 6 to obtain a fourth algal suspension which is more concentrated than said third algal suspension,
preferably wherein step b2) comprises introducing said algal suspension into said first flow path in an introduction stage during which algae are forced to be retained in said first flow path, and wherein said introducing of the algal suspension at said pressure difference results in accumulation of algae on a first side of said membrane and introduction of water in said second flow path; and wherein recovering the accumulated algae from said first side of the membrane is performed in a subsequent stage which comprises releasing the accumulated algae from said first side of said membrane with a liquid medium in an amount less than the amount of water flowed across the membrane in said introduction stage to obtain an algal suspension which is concentrated compared to the algal suspension introduced into said first flow path.

11. A method for producing algal products comprising:
a) cultivation of algae in an aqueous culture medium to provide an algal suspension;
b) concentrating said algal suspension in accordance with the method of claims 1 to 6 to obtain an algal suspension which is more concentrated compared to the algal suspension of a); and
c) extracting algal products from the concentrated algal suspension obtained in b).

12. A system for producing a concentrated algal suspension, comprising
a bioreactor configured to cultivate a first algal suspension,
at least one first device having at least two flow paths separated by a membrane wherein a first flow path on one side of said membrane is configured to receive said first algal suspension from said bioreactor,
means to apply a pressure difference over said membrane to enable flow of water across the membrane to a second of said flow paths; and
means to recover algae from said first flow path as a second algal suspension which is more concentrated than said first algal suspension.

13. The system according to claim 12, which comprises a plurality of first devices arranged in parallel.

14. The system according to claim 12 or 13, wherein said means to apply a pressure difference over said membrane to enable flow of water across the membrane to a second of said flow paths is selected from:
- a means for applying suction onto the second flow path; and
- a means for introducing said first algal suspension into the first flow path under pressure.

15. The system according to any of the claims 12 to 14, comprising
a first bioreactor configured to cultivate said first algal suspension,
at least one first device having at least two flow paths separated by a membrane, wherein a first flow path on one side of said membrane is configured to receive said first algal suspension from said first bioreactor,
means to apply a pressure difference over said membrane of said at least one first device to allow flow of water across the membrane to a second of said flow paths; and
means to recover algae from said first flow path of said at least one first device as a second algal suspension which is more concentrated than said first algal suspension;
a second bioreactor configured to receive said second algal suspension from said at least one first device, and which is further configured to cultivate said second algal suspension under different cultivation conditions than in the first bioreactor to provide a third algal suspension;
at least one second device having at least two flow paths separated by a membrane, wherein a first flow path on one side of said membrane is configured to receive said third algal suspension from said second bioreactor,
means to apply a pressure difference over said membrane of said at least one second device to allow flow of water across the membrane to a second of said flow paths; and
means to recover algae from said first flow path of said at least one second device as a fourth algal suspension which is more concentrated than said third algal suspension.
